# EUROPEAN PATENT APPLICATION

(11) **EP 2 626 008 A1**
(43) Date of publication of application: **14.08.2013**
(21) Application number: 12154912.5
(22) Date of filing: 10.02.2012
(51) Int. Cl.: A61B 6/04

(54) **Device for fixating a body part and a method thereof**

(71) Applicant: Teamolmed Syd AB, 551 85 Jönköping (SE)
(72) Inventor: Ceder, Michael, 561 35 Huskvarna (SE)
(74) Representative: Bokinge, Ole

(57) **Abstract**

The present invention relates to a device (1) for fixating body part comprising a sleeve member (2) of a hermetically impermeable material (3) to be placed around a body part (10), having at least one open end (5), wherein at least one sealing means (4) is provided in order to achieve a tight seal between the sealing means (4) and the body part (10), the device further comprising a valve (6) to be arranged to facilitate a vacuum to be obtained between the sleeve member (2) and the body part.

The vacuum is thus providing fixation to a body part (10) to aid medical scanning of a body part (10).

The present invention also relates to a method for fixating a body part (10).

## Description

### Field of the Invention

The present invention relates to a device and method for fixating a body part, and especially to a device and method for fixating a body part in order to facilitate 3d-scanning of the body part.

### Background of the Invention

3d-scanning of a body part is commonly used in the making of prostheses and orthoses. An orthosis is a device applied to a human body part to control or enhance movement or to prevent bone movement or deformity, and can be used by patients suffering from various conditions that cause for example spasms or a misdirected body part. Prosthesis is used for compensating for or replacing a missing body part.

During the making of a custom made orthoses or prostheses, the patient's body part to which the orthosis or prosthesis is to be fitted is measured and modelled. The model is usually a physical model which serves as a base on which an orthosis or prosthesis is made. The model can be made in various ways. A conventional way is to make a negative plaster cast of the body part. From the plaster cast, a positive model of the body part is made, and with the positive model as a pattern, an orthosis or prosthesis is formed. The plaster casting procedure may be time consuming and fairly complicated since the procedure involves wet plaster and long stiffening times. The plaster cast may also be sensitive to any impact that may cause the plaster cast to break, and thus has to be repaired or possibly a completely new cast may be required.

Another commonly used method is to scan the body part to receive a 3-dimensional digital image of the body part. The digital image is the basis for the fabrication of a model of which the orthosis or prosthesis is formed. This is a more time efficient procedure, which does not depend on the quality of the plaster nor the resulting cast, also it requires less involvement from the patient since the scanning procedure may be significantly quicker.

The scanning procedure may be performed by using a stationary scanning apparatus, or possibly a portable scanning apparatus. Depending on the type of procedure, the requirements may differ.

During a scanning procedure, it is desirable that the scanning camera obtains an as unbroken view of the body part as possible, since any obstructing objects may reduce the image quality. Also, it is required that the patient holds the body part as static as possible in a normal position for the scan to produce an accurate image. Any movement from the patient may result in a poor image, and the scanning may be repeated until the scanning is successful. To hold the body part static in a normal position during a longer period of time may be nearly impossible for many patients that suffer from neurological conditions which may involve spastic symptoms or misdirected limbs. Because of this, a normal position may only be obtained by having staff assisting by holding the patient in a still position or by applying certain force to the body part, and hence numerous scans may be required in order to produce an image of acceptable quality of the body part. This can be very time consuming, require numerous assisting personnel and may cause stress and discomfort for the patient. One way of facilitating the fixation of the patient's body part is to let the patient rest the body part to be scanned on a transparent support. The body part may then further be mounted to the support, for instance by strapping. However, since the best result is achieved by having an unbroken view of the body part, this procedure requires supporting and strapping arrangements that does not disturb the view of the scanner as much. Also, any forceful retaining of a patient, such as strapping, may cause stress and discomfort, especially for patients with nervous conditions.

Consequently, there is a need for a solution to the problem in obtaining fixation of a body part to be scanned. Furthermore, there is a need for a solution to the problem in obtaining fixation of a body part to be scanned, without the need of any external strapping means that may cause distress and discomfort for a patient, and may obstruct the view of the scanning camera.

### Summary of the Invention

It is an object of the present invention to provide a device that provides a fixating effect, when applied on a body part, in order to facilitate medical handling of patients, especially patients suffering from neurological conditions or physical disorders that lead to misdirected limbs or spastic symptoms or such.

A further object is to provide a device that obtains fixation of a body part to be scanned, wherein the device is adapted for 3d-scanning and reduces the risk of obstructing the vision for the scanning camera.

According to a first aspect of the invention, these and other objects are achieved by a device for fixating a body part comprising a sleeve member of a hermetically impermeable material. The sleeve member is adapted to enclose a body part and comprises at least one open end. Further, the sleeve member comprises at least one sealing means arranged around the circumference of the open end and is adapted to provide an air tight seal towards the body part. Further, the device comprises a valve adapted to enable a vacuum between the sleeve member and the body part.

There may be one sealing means at each open end of the sleeve member. The sealing means may provide that no air accidentally enters the fixating device while being under a vacuum. The other ends and edges of the sleeve member may be sealed together with glue or a weld or such so that the fixating device is substantially hermetically impermeable. If the sleeve member is provided with one open end, it may be formed as a bag and all other ends and edges being permanently sealed from the air. This may allow the device to be used successfully on body parts that do not extend through the entire sleeve member, such as a hand or a foot, where there is need for only one sealing means against the body part.

The fixating effect may thus be obtained with the combination of a hermetically impermeable material that may enclose and be sealed towards the body part from the surrounding environment, and a vacuum being applied between the material and the body part so that the material may form a close fit which provides a stiffening effect of the body part in the required position. While the vacuum is applied, the medical procedure may be performed as required.

The sealing means at the open end of the sleeve member may be adapted to seal off any external air, and to provide a seal that enables a vacuum to be applied to the device. The vacuum may be produced using an electric vacuum pump. An external vacuum pump may be used, or if there is access to such, for instance in a hospital environment, centrally controlled vacuum may be used. The valve may be adapted to fit the vacuum nozzle, which may vary depending on the type of valve used, or depending on implemented standards in different countries. The valve may also be of different type and performance. However, it may be adapted so that no air accidentally enters the fixating device when the vacuum is applied.

The stiffening effect produced by the vacuum may fixate the body part in a desired position, for the body part to stay static during the scanning procedure. Since the body part may be fixated due to the vacuum, there is no need for the interference of strapping or holding during the scanning procedure, which may allow the patient to feel less trapped and hence reducing the stress and discomfort for the patient. Further, the scan may be performed with any type of scanning camera available, which may be of a stationary type or a portable type. Depending on the type used, the procedure may differ, for instance the scanning camera may be moved around and along a body part to obtain a full image. Hence, since the body part may be free, there is a reduced risk that there may be obstacles, like strapping, that obstructs the view of the scanning camera, which may increase the chance of the image to be accurate after a first scanning cycle. Consequently, a scanning cycle may require less time since the position of the body part remains static until the seal is loosened and the vacuum is released. This may be an advantage since the risk of having to repeat the procedure will be reduced, and thus the accumulated production time per orthosis or prosthesis may be significantly reduced.

According to one embodiment of the invention, the sleeve member may be of a transparent hermetically impermeable material.

Since the fixated body part may be scanned, a good result may be obtained if the scanning camera has a clear view of the body part. The fixating device may therefore be substantially transparent when used for the purpose of 3d-scanning so that the camera may scan through any uneven surface of the material. This enables the image to be reproduced as natural as possible. Further, it may enable an operator of the scanning of the body part to see any wounds on the body part, which wounds may be relevant for the scanning and the orthosis or prosthesis fabrication.

According to one embodiment of the invention, the hermetically impermeable material may be a film of a plastic material, such as polyvinyl chloride (PVC) or polyethylene (PE) or the like. A sufficient stiffening effect of the sleeve member for the body part to become substantially immobile during the scanning procedure may thereby be achieved. The material may preferably be thin, so that the true contour of the body part may be shown when the vacuum is applied to the device, providing a semi rigid material. The material may also be flexible enough to easily form around the body part, but rigid enough to provide adequate stiffness when the vacuum is applied. This may enable the body part to be reproduced as true as possible and facilitates an accurate model of the body part.

According to one embodiment of the invention, the sleeve member may be formed from a single layer of the hermetically impermeable material.

This is due to the fact that it may be advantageous to use a sleeve member containing as little material as possible, since less material may reduce the risk of creasing and wrinkling around the body part. Hence, the image of the body part may be more naturally reproduced. Also, since the vacuum is being produced between the fixating device and the body part, there may be no need for any additional layers of material.

According to one embodiment of the invention, the sealing means may comprise a sealing ring of a soft flexible material arranged to face an inner surface of the sleeve member at said at least one open end, and a pressing member adapted to apply a pressure towards the body part so that the sealing ring may form a tight seal against the body part.

By letting the sealing ring consist of a soft flexible material, it may be allowed to form closely around the body part in order to provide a tight seal that allows the vacuum to be sufficiently applied, and also feel soft and comfortable against the skin of the patient. The pressing member may enable a pressure towards the body part to be applied. This pressing member may secure a static pressure while the sealing ring, which is placed between the pressing member and the body part, may provide a seal against the body part while the vacuum is applied, in order to prevent air leakage. The sealing ring may be fixed to the sleeve member, or a separate part arranged between the sleeve member and the body part when the device is arranged onto a body part of a patient. Further, the pressing member may be attached to the sleeve member, or a separate part arranged outside the sleeve member and adapted to provide a pressure towards the body part.

According to one embodiment, the pressing member may be of an elastic type band or tubing that may be manually tightened and released as required. It may also be of a rigid type belt or string. The pressing member may also be of tubing or a channel that obtains a pressure by being inflated or filled with a fluid, and thus pressing against the body part. However, any type pressing member that sufficiently provides static pressure towards the body part may be possible.

According to one embodiment of the invention, the sealing ring may be of a soft elastomer like a rubber material or a synthetic rubber. By using a soft elastomer like rubber, a sealing effect against human skin may be easier obtained since it allows certain retraction against the body part, and thus enabling a sufficient seal.

According to one embodiment, the fixating device may be provided with two open ends. Further, the device may comprise a plurality of open ends, each provided with a sealing means. This type of fixating device may be required when a body part like a knee or an elbow needs to be fixated. The fixating device may then be slipped over the body part, which extends through the sleeve member, and sealed against the skin at two ends, leaving the part to be fixated in between the two sealed ends.

According to an embodiment of the invention, the fixating device may further comprise at least one suspending element.

This may be used when the body part needs additional support to stay in a required position. These suspending elements may be a type of hole, loop, clip or the like that allows the body part to be stretched or suspended in a required position onto a support by a fastening element such as a hook or button. Another solution may be using rings, straps, or any other appropriate suspending element, and instead of leaning the body part on a support it may be considered that the fixated body part may be secured in a hanging position to facilitate scanning. The suspending element may be placed so that the view of the scanning camera is clear.

According to one embodiment of the invention, the at least one suspending element may be arranged on a part of the sleeve member not affected by a vacuum between the sleeve member and the body part.

By arranging the suspending elements so that they are unaffected of the vacuum, this may prevent any accidental perforation of the sleeve member when suspending the body part. The suspending elements may be placed along any surface of the fixating device that may be required.

According to one embodiment of the invention, the valve may be a non-return valve.

If the valve is not a non-return valve, the air must be sucked through the valve constantly during the fixating/scanning procedure. This may be advantageous if the sealing is not completely tight. However, if the sealing provides for it, and if the valve is a non-return valve, the application of vacuum in the sleeve member towards the body part may be terminated when the vacuum is achieved. One way of releasing the vacuum may then be to release the sealing.

According to one embodiment, an air gap means may be provided in the sleeve member in connection with the valve.

When vacuum is applied through the valve, the sleeve member will be drawn towards the body part. It may then occur that a part of the sleeve member adjacent to the valve may be drawn to the body part such that an airtight sealing around the valve is formed, preventing vacuum for being achieved in a remote section of the sleeve member from the valve. Thereby, an air gap may be needed between the sleeve member and the body part, at least proximate the valve, to prevent an airtight sealing to be formed. The air gap means may in one embodiment be a fabric hose or the like, slipped onto the body part. Such hose may prevent the sleeve member from forming an airtight sealing towards the body part. Air transportation may always be possible through the air gap means even when the sleeve member is drawn towards the body part. The air gap means, for instance in the form of a hose, may be thin enough to not interfere with the scanning operation of the shape of the body part.

Alternatively, the air gap means may be a woven rope or string, or a perforated tube or the like, arranged inside the sleeve member to prevent the sleeve member from forming an airtight sealing with itself. Hence, if the valve may be provided at a corner or end part of the sleeve member, not in a section directly in contact with the body part, the sleeve member may, when vacuum is applied, be drawn to itself and form an airtight sealing, thereby preventing vacuum to be applied in other sections of the sleeve member. By providing an air gap means in the form of a rope or string extending from the valve towards a remote section of the sleeve member from the valve, an airtight sealing of the sleeve member to itself may be prevented. During a 3d-scanning operation, computer software used for the scanning may be programmed to ignore the presence of the air gap means. For instance, if the air gap means is a rope, string or tube, the rope, string or tube may be in a colour that the software may be programmed to ignore.

According to a second aspect, the present invention provides a method for fixating a body part. This is obtained by first arranging a hermetically impermeable fixating device with at least one open end to enclose a body part. Further, the method comprises the steps of sealing the at least one open end of the fixating device towards the body part, and applying vacuum between the fixating device and the body part.

Depending on the body part on which the fixating device is to be used, it may vary how the device is arranged. However, the fixating device may completely surround the body part to be fixated, and the part to be scanned may be visible to the camera through the hermetically impermeable material. After applying the fixating device, the fixating device may be sealed towards the body part in order to enable the vacuum to be produced. The sealing may be done with aid of a sealing means at the open end or ends towards the body part so that an air tight seal between the fixating device and the skin is obtained. Depending on the type of sealing means to be used, this step may be adapted accordingly. It may be by tightening a pressing member, like an elastic band or a belt, tying a string or a strap, or something of the like, towards a sealing ring that is placed between the pressing member and the body part, to obtain a sealing effect. After sealing, in order to prepare the body part for fixating in the correct position, it may be required to straighten the body part by holding it briefly in a desired position until the vacuum has been properly applied. Subsequently, there is a step of applying a vacuum to the fixating device to obtain a stiffening effect of the body part. The vacuum may provide adequate stiffness to the body part and may be adapted accordingly until the expected result is gained. The vacuum may be obtained through a valve in the fixating device. When the medical scan, or any other medical procedure required, has been performed, the vacuum may be released by for example lifting the seal, or possibly venting air in through the valve, if the valve is of a type that allows that. An alternative could be having a second valve arranged, in order to release pressure. Releasing the vacuum may also be necessary if the body part was fixated in a faulty position, which may involve subsequent fixating attempts, by repeating the steps above. It may also be possible to apply the vacuum with a sort of frequency, and therefore the vacuum may be applied and released by a timer or clock or the like. In a further alternative, if the sealing of the open end is not completely sealed, the vacuum may be provided constantly during the fixation through the valve. The valve does thereby not have to be a non-return valve.

The method may involve intermediate steps in order to achieve a required scanning result. It may be required to further improve positioning of the body part, by using the suspending elements, in order to obtain a desired position before applying vacuum, alternatively after applying vacuum, but before the medical procedure.

### Brief Description of the Drawings

For a better understanding of the invention reference will now be made, by way of example, to the accompanying drawings, in which:
Fig. 1 a is a schematic view of a fixating device according to an embodiment of the invention.
Fig. 1b is a schematic view of a fixating device according to an embodiment of the invention.
Fig. 1c is a schematic view of a fixating device according to an embodiment of the invention.
Fig. 1d is a schematic view of a fixating device being applied onto a patient according to an embodiment of the invention.
Fig. 2 is a schematic cross-sectional view of a sealing means according to an embodiment of the invention.
Fig. 3 is a schematic view of a fixating device according to an embodiment of the invention.
Fig. 4 is a schematic view of a fixating device according to an embodiment of the invention.

### Description of Exemplary Embodiments of the Invention

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

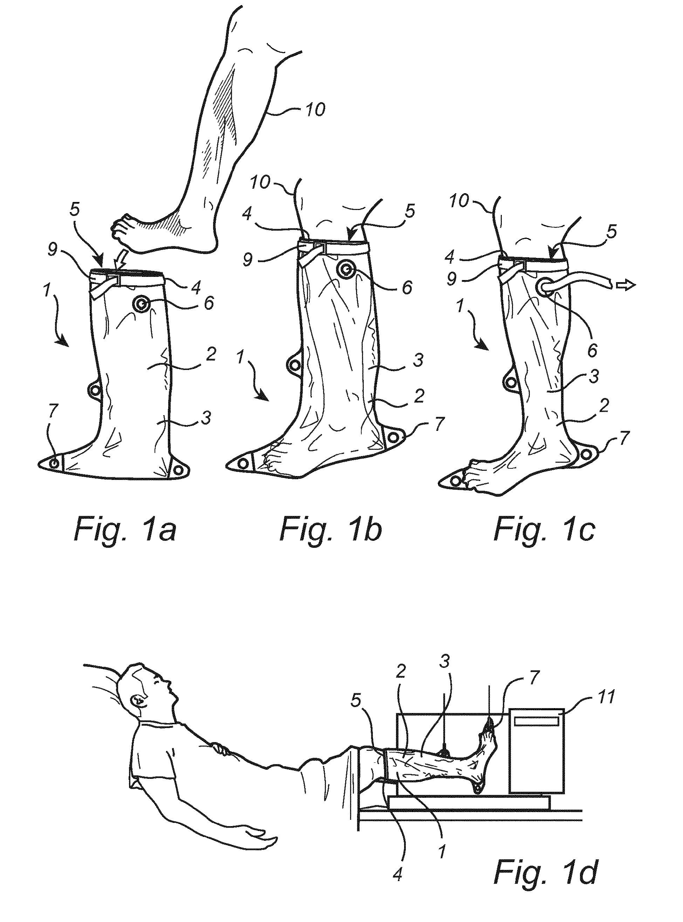

In Fig. 1a-c, an embodiment of a fixating device 1 for fixating a body part 10, adapted to fit a foot and ankle, is shown. The exemplary embodiment is shaped as a boot or a sock, having a sleeve member 2, having one open end 5 where a sealing means 4 is provided, adapted to seal off any external air. The sleeve member 2 is of a transparent, hermetically impermeable material 3, suitable for 3d-scanning and to enable a vacuum, and is equipped with a valve 6, adapted to enable any air to be removed from the volume between the fixating device and the body part 10 in order to obtain a vacuum. The valve 6 may be, as shown, arranged directly on the sleeve member 2, but it may also be possible that the valve 6 is placed somewhere else in connection to the sleeve member, for instance along a tube that may be connected to the sleeve member 2. However, any other arrangements in obtaining a vacuum may be possible. The valve 6 may be a non-return valve.

The fixating device 1 may be provided with suspending elements 7, which may be in the shape of holes in order to place the body part 10 in for example a hanging position. By having such suspending elements 7, it is possible to provide additional positioning of the body part 10 before a scanning operation. This may be necessary due the scanning apparatus' 11 position or possibly if the patient is required to obtain a lying position.

In Fig. 1b, the fixating device 1 is applied onto a patient's body part 10, the foot, wherein the foot is placed and the fixating device 1 is sealed around the leg with the sealing means 4 to prepare for the vacuum to be applied.

In Fig. 1c, the fitted fixating device 1 is being exposed to a vacuum, the air being removed through a valve 6. The vacuum provides a stiffening effect on the body part 10 and may keep the body part 10 static during a medical task, for example a 3d-scan. To enable sufficient vacuum to be produced, and to obtain a tight stiffening effect, a hermetically impermeable material 3 should be used. The material 3 should also be substantially transparent in order to obtain a clear view of the body part when a scan is being performed. By letting the fixating device 1 maintain a fixation of a body part 10 without the need of any external strapping, this solution may be suitable for any type of scanning apparatuses 11. A scanning apparatus 11 may be of a stationary type or portable type, wherein the scanning apparatus may be rotated around a body part 10 without obstruction in order to obtain a full scan.

The fixating device 1 should be adapted to fit comfortably over the body part 10 so that there is as little excess material as possible, since excess material may cause creases and wrinkles when the vacuum is applied, which may cause disturbances to the scanned image. Because of this, it is suitable that the sleeve member 2 is of a single layer material. Also, the fixating device 1 may be available in various sizes and shapes to fit patients of any age and size, as well as different body parts 10. A fixating device 1 should generally be sized so that the part of the body to be scanned is adequately visible. Between the sleeve member 2 and the body part 10, an air gap means such as a fabric hose (not shown) may be arranged. Such air gap means would facilitate the vacuum application and enhance the fixating operation. The air gap means may be provided in a way that it does not interfere with the scanning operation of the body part 10.

Fig. 1d shows an exemplary set-up of a patient's foot in a hanging position in front of a scanning apparatus 11, showing the use of additional suspending elements 7. A hanging position may be necessary in the case a scanning apparatus 11 is placed in a relatively high position, or if a patient requires a certain set-up due to immobileness or such. However, the suspending elements 7 may be used for any other purpose required, such as to stretch a fixated body part 10 along a resting support. The suspending elements 7 may also be of any size, shape or form that may be required, such as a hook, strap, hole or clip. The suspending elements 7 may be placed so that they do not constitute an part of the fixating device 1 that is exposed to vacuum, but may instead be arranged to the be unaffected by the vacuum. This may be preferred in order to avoid accidental penetration, and thus damaging the sleeve member 2.

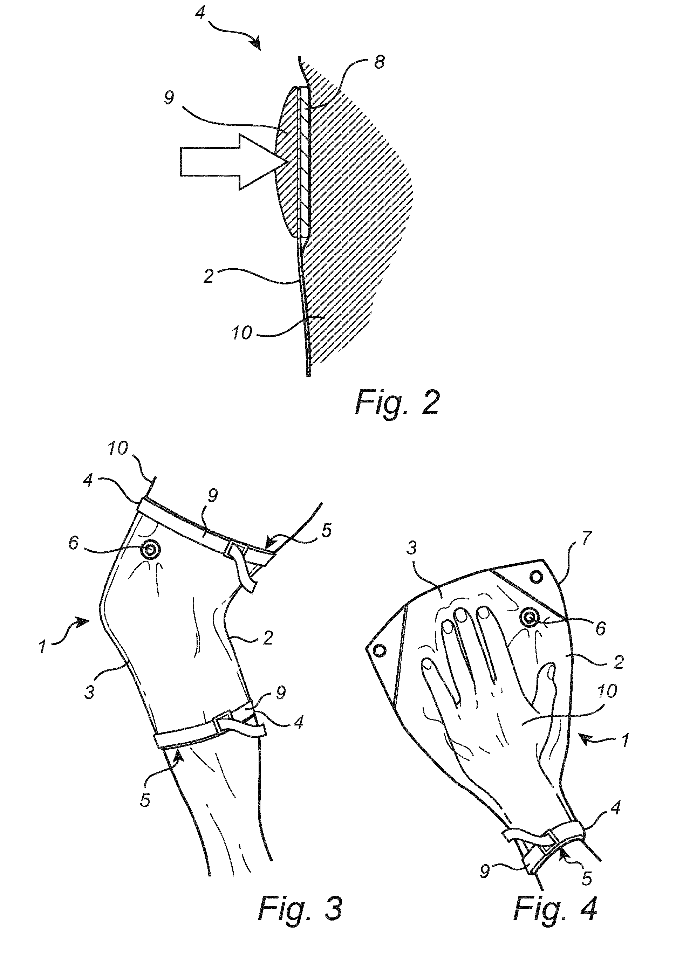

Fig. 2 shows an example of a sealing means 4, wherein a sealing ring 8 is placed facing the body part, and a pressing member 9 applies a static pressure onto the sealing ring 8 to obtain a hermetic seal. As shown, the sleeve member 2 may placed between the sealing member 8 and the pressing member 9. The material used on the sealing ring 8 should preferably be a soft elastomer type material that allows close contact to the skin and enhances a sealing effect and provides comfort and softness against the skin. However, any type of material that allows adequate sealing effect may be used. The pressing member 9 may be any type of arrangement that provides a static pressure onto the sealing ring 8, such as an elastic band, or possibly a belt or a string that may be manually tightened and released as required. It may also be of a tube or channel or the like that obtains a pressure by being inflated or filled with a fluid, and thus releases the pressure by being deflated or emptied of fluid. The sealing means should generally be sized adapted for the purpose so intended sealing effect is achieved. It is also possible that the sealing ring 8 and the pressing member 9 are integrated into one unit, or may be of the same piece of material. The sealing means 4 may not provide a completely airtight sealing towards the body part 10. In that case, a constant vacuum suction is provided through the valve 6 by a vacuum pump.

In Fig. 3, a fixating device 1 adapted for use on a knee is shown, showing an arrangement with two open ends 5, having one sealing means 4 at each open end 5. A body part 10 extending through the sleeve member 2 may thereby be fixed. This type of arrangement may be necessary for other parts of a human body that may extend through a sleeve member 2, such that the body part may be fixed when the two open ends 5 of the sleeve member 2 are sealed.

As shown in Fig. 4, a fixating device 1 for a hand may be shaped as a regular plastic bag, having one open end 5. An alternative could be letting a fixating device for a hand be in the shape of a glove. In addition to the exemplary body parts 10 shown, the fixating device 1 may be adapted for use on a shoulder, leg, torso or any other part of the body that may need fixating.

As shown in Fig. 1a-4, the fixating device 1 is of a transparent material adapted for 3d-scanning or possibly photographing. However, should transparency be of less importance, the fixating device 1 may be of any other colour that may be suitable for that purpose. The fixating device 1 may be for fixating a body part 10 in general, but it is possible for the fixating device 1 to be used for other purposes. It is possible to use the fixating device 1 when it is required to apply a pressure onto a body part 10, by vacuum. Such use could be in order to increase blood flow, or restrict blood, or any other use that require pressurising a body part 10. It would also be possible to arrange the fixating device 1 so that pressure is applied and released in cycles, for instance in a pulsating manner or any other frequency by being connected to a timer or such to obtain a required result.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

**1.** A device (1) for fixating a body part (10) **characterized in that** said device (1) comprises
a sleeve member (2) of a hermetically impermeable material (3) adapted to enclose a body part (10), said sleeve member (2) having at least one open end (5), said sleeve member (2) comprising at least one sealing means (4) arranged around the circumference of said open end (5) and adapted to provide an air tight seal towards the body part (10), said device (1) further comprising
a valve (6) adapted to enable vacuum between said sleeve member (2) and the body part (10).

**2.** A device (1) for fixating a body part (10) according to claim 1,
wherein said sleeve member (2) is of a transparent hermetically impermeable material (3).

**3.** A device (1) for fixating a body part (10) according to claim 1 or 2,
wherein said hermetically impermeable material (3) is a film of a plastic material, such as polyvinyl chloride (PVC), or polyethylene (PE) or the like.

**4.** A device (1) for fixating a body part (10) according to any of the preceding claims, wherein said sleeve member (2) is formed from a single layer of said hermetically impermeable material (3).

**5.** A device (1) for fixating a body part (10) according to any of the preceding claims, wherein said sealing means (4) comprises a sealing ring (8) of a soft flexible material arranged to face an inner surface of the sleeve member (2) at said at least one open end (5) and a pressing member (9) adapted to apply a pressure towards the body part (10) so that the sealing ring (8) forms a tight seal against the body part (10).

**6.** A device (1) for fixating a body part (10) according to claim 5,
wherein said soft flexible material is an elastomer, such as rubber or the like.

**7.** A device (1) for fixating a body part (10) according to any of the preceding claims, wherein said fixating device (1) is provided with two open ends (5).

**8.** A device (1) for fixating a body part (10) according to any of the preceding claims, further comprising at least one suspending element (7), such as a hole, loop, hook, button, ring, strap, clip or the like.

**9.** A device (1) for fixating a body part (10) according to claim 8,
wherein said at least one suspending element (7) is arranged on a part of the sleeve member (2) not affected by a vacuum between the sleeve member (2) and the body part (10).

**10.** A device (1) for fixating a body part (10) according to any of the preceding claims, wherein said valve (6) is a non-return valve.

**11.** A device (1) for fixating a body part (10) according to any of the preceding claims, wherein an air gap means is provided in the sleeve member in connection with the valve (6).

**11.** A method for fixating a body part (10) comprising the steps of:
arranging a hermetically impermeable fixating device (1) with at least one open end (5) to enclose a body part (10),
sealing the at least one open end (5) of the fixating device (1) towards the body part (10), and
applying vacuum between the fixating device (1) and the body part (10).

**12.** A method for fixating a body part according to claim 11, wherein the step of applying vacuum comprises removing air from the fixating device through a valve (6).

**13.** A method for fixating a body part according to claim 11 or 12,
wherein the step of sealing the at least one open end (5) comprises applying a pressure on a sealing ring (8) provided at an inner surface of the fixating device (1) towards the body part (10).

**14.** A method for fixating a body part (10) according to any of the claims 11-13, wherein the method further comprises the step of suspending the fixating device (1) and the body part (10) by means of at least one suspending element (7) arranged on the fixating device (1).
